# EUROPEAN PATENT SPECIFICATION

(11) **EP 4 135 478 B1**
(45) Date of publication and mention of the grant of the patent: **25.03.2026**
(21) Application number: 22190231.5
(22) Date of filing: 12.08.2022
(51) Int. Cl.: H05B 3/28, H05B 3/26, A24F 40/46

(54) **ATOMIZING ELEMENT, ATOMIZER, AND ELECTRONIC ATOMIZING DEVICE**
ZERSTÄUBUNGSELEMENT, ZERSTÄUBER UND ELEKTRONISCHE ZERSTÄUBUNGSVORRICHTUNG
ÉLÉMENT D'ATOMISATION, ATOMISEUR ET DISPOSITIF D'ATOMISATION ÉLECTRONIQUE

(30) Priority: 13.08.2021 CN 202110931780
(43) Date of publication of application: 15.02.2023
(73) Proprietor: Shenzhen Smoore Technology Limited, Shenzhen, Guangdong 518102 (CN)
(72) Inventor: LV, Hongxia, Shenzhen (CN); JIANG, Zhenlong, Shenzhen (CN); LI, Pei, Shenzhen (CN)
(74) Representative: Herrero & Asociados, S.L.

(56) References cited:
- WO-A1-2020/238607
- CN-A- 110 720 675
- CN-C- 100 376 857

## Description

### TECHNICAL FIELD

The present disclosure relates to the technical field of electronic atomizing devices, and in particular to an atomizing element, an atomizer, and an electronic atomizing device.

### BACKGROUND

An electronic atomizing device is a device which may heat an aerosol-generating substance to generate an aerosol. An atomizing element of the electronic atomizing device usually includes a ceramic substrate and a metal film arranged on an atomizing surface of the ceramic substrate. After being energized, the metal film heats the aerosol-generating substance close to the atomizing surface.

The metal film is prone to an oxidation failure during a sintering process and an atomizing process. Especially when an oil supply is insufficient during the atomizing process, the metal film is extremely easy to have a failure. As a result, a stability and a life of the electronic atomizing device are severely affected. Therefore, a heating metal film including a precious metal material is used in an existing atomizing element, and the precious metal accounts for a relatively high proportion in the heating metal film. The precious metal is usually a material such as gold, platinum, or the like. However, when the oil supply is insufficient, the heating metal film with the precious metal in a high proportion is prone to over-burning. Precious metal particles in the heating metal film are prone to agglomerating, which in turn leads to the failure of the metal film. In this way, the stability and a life of the atomizing element may be reduced. In addition, a cost of the precious metal is relatively high, such that a cost of the atomizing element may be relatively high.

WO2020238607A1 discloses an atomization member and an electronic cigarette. The atomization member includes a porous substrate, a dielectric layer, a resistance layer, and a modification layer. The porous substrate is provided with an atomization surface; the dielectric layer, the resistance layer, and the modification layer are arranged on the atomization surface; and the dielectric layer, the resistance layer, and the modification layer are sequentially stacked and formed on the porous substrate. The porous substrate is configured to absorb an aerosol forming substrate and conduct the aerosol forming substrate onto the atomization surface. The resistance layer is configured to convert electric energy into heat energy and heat and atomize the aerosol forming substrate on the atomization surface. The dielectric layer provides a substrate for the formation of the resistance layer on the porous substrate. The modification layer causes the resistance layer to be modified in terms of preset expected performance. The resistance layer is tightly combined with the atomization surface so as to prevent the resistance layer from failing due to heating without e-liquid.

CN110720675A discloses a heating body, a preparation method of the heating body, and an electronic cigarette. The heating body includes a plurality of porous metal layers in stacking arrangement, and the hole diameters of the plurality of porous metal layers are gradually reduced along the stacking direction. The heating body can play an effective preheating role on tobacco juice, so that the atomizing is more sufficient, the generated smoke amount is larger, then the smoke mouthfeel is better. In addition, various tobacco juices can be matched, and especially, excellent smoking experience can also be obtained for high-viscosity tobacco juice.

### SUMMARY

An atomizing element, an atomizer, and an electronic atomizing device are provided in the present disclosure.

To resolve a technical problem described above, a first technical solution provided in the present disclosure is to provide an atomizing element. The atomizing element includes a substrate and a heating film. The substrate includes an atomizing surface. The heating film is arranged on the atomizing surface and is capable of heating and atomizing an aerosol-generating substance on the atomizing surface in response to being energized.

The heating film includes a metal heating layer and an inorganic protection layer stacked with the metal heating layer. The inorganic protection layer is arranged on a surface of the metal heating layer away from the substrate.

The metal heating layer includes at least two sequentially stacked sub-metal layer, and any two adjacent sub-metal layers have different compositions.

In some embodiments, the substrate is a porous substrate, or the metal heating layer and/or the inorganic protection layer are porous structures.

In some embodiments, the inorganic protection layer has lipophilicity and/or hydrophilicity.

In some embodiments, the metal heating layer includes a first sub-metal layer and a second sub-metal layer stacked with the first sub-metal layer. A material of the first sub-metal layer includes a NiCr alloy or a 316L stainless steel, and a material of the second sub-metal layer includes TiZr alloy.

In some embodiments, the metal heating layer is in direct contact with the substrate through the second sub-metal layer.

In some embodiments, the number of the first sub-metal layer is at least two, and the number of the second sub-metal layer is at least two.

In some embodiments, a total thickness of the first sub-metal layer is in a range of 1 µm to 20 µm, a total thickness of the second sub-metal layer is in a range of 0.5 µm to 5 µm, and a thickness of the inorganic protection layer is in a range of 0.1 µm to 5 µm.

In some embodiments, a shape of the heating film is an S shape, a circle, an ellipse, a semicircle or a ring; or a shape of the metal heating layer is an S shape, a circle, an ellipse, a semicircle, or a ring.

In some embodiments, the atomizing element further includes two electrodes, one of two electrodes is arranged on a first side of the metal heating layer, the other one of the two electrodes is arranged on a second side of the metal heating layer opposite to the first side of the metal heating layer, and the two electrodes are electrically connected to the metal heating layer. The inorganic protection layer covers a portion of the metal heating layer located between the two electrodes.

In some embodiments, the heating film is a rectangle, the one of the two electrodes is arranged on a first side of the rectangle, the other one of the two electrodes is arranged on a second side of the rectangle opposite to the first side of the of the rectangle, and a hollow which does not generate heat is arranged in the heating film.

In some embodiments, the hollow includes first hollows and a second hollow, a first one of the first hollows is located on a third side of the rectangle, a second one of the first hollows is located on a fourth side of the rectangle opposite to the third side of the rectangle, and the third side of the rectangle is vertical to the first side of the rectangle. The first hollows are fan-shaped. The second hollow is located on a middle of the heating film, and a shape of the second hollow is an ellipse or a circle.

In some embodiments, a modification layer is further arranged on an atomizing surface of the porous substrate, and a thickness of the modification layer is in a range of 50 µm to 300 µm.

In some embodiments, the modification layer includes a composition in parts by mass of 56 to 67.5 parts of silicon dioxide, 12 to 18 parts of aluminum oxide, and 2.8 to 5.5 parts of lithium oxide.

To resolve the technical problem, a second technical solution provided in the present disclosure is to provide an atomizer. The atomizer includes a liquid storage chamber and an atomizing element. The liquid storage chamber is configured to store an aerosol-generating substance, and the atomizing element is the atomizing element described above.

To resolve the technical problem, a third technical solution provided in the present disclosure is to provide an electronic atomizing device. The electronic atomizing device includes a battery assembly and an atomizer. The battery assembly is configured to supply power to the atomizer, and the atomizer is the atomizer described above.

### BRIEF DESCRIPTION OF THE DRAWINGS

To describe technical solutions in the embodiments of the present disclosure more clearly, accompanying drawings required for describing the embodiments are introduced briefly in the following. Apparently, the accompanying drawings described in the following show only some embodiments of present disclosure.
FIG. 1 is a schematic structural diagram of an electronic atomizing device according to some embodiments of the present disclosure.
FIG. 2 is a schematic structural diagram of an atomizer according to some embodiments of the present disclosure.
FIG. 3 is a schematic structural diagram of an atomizing element according to a first embodiment of the present disclosure.
FIG. 4 is a schematic structural diagram of the atomizing element according to a second embodiment of the present disclosure.
FIG. 5 is a schematic structural diagram of the atomizing element according to a third embodiment of the present disclosure.
FIG. 6 is a scanning electron micrograph of a heating film prepared by directly arranging a NiCr alloy metal layer or a 316L stainless steel metal layer on a substrate.
FIG. 7 is a scanning electron micrograph of the atomizing element according to an embodiment of the present disclosure.
FIG. 8 is a schematic structural diagram of the atomizing element according to a fourth embodiment of the present disclosure.
FIG. 9 is a schematic structural diagram of the atomizing element according to a fifth embodiment of the present disclosure.
FIG. 10 is a schematic structural diagram of the atomizing element according to a sixth embodiment of the present disclosure.
FIG. 11 is a schematic structural diagram of the atomizing element according to a seventh embodiment of the present disclosure.
FIG. 12 is a flowchart of a preparation method for the atomizing element according to the first embodiment of the present disclosure.
FIG. 13 is a flowchart of a preparation method for the atomizing element according to the second embodiment of the present disclosure.
FIG. 14 is a flowchart of a preparation method for the atomizing element according to the third embodiment of the present disclosure.
FIG. 15 is a flowchart of a preparation method for the atomizing element according to the fourth embodiment of the present disclosure.

### DETAILED DESCRIPTION

The technical solutions in the embodiments of present disclosure are clearly and completely described in the following with reference to the accompanying drawings in the embodiments of present disclosure. Apparently, the described embodiments are merely some rather than all of the embodiments of present disclosure.

In the following descriptions, to illustrate rather than limit, specific details such as a particular system structure, an interface, and a technology are provided to make a thorough understanding of present disclosure.

In the present disclosure, the terms "first", "second", and "third" are used merely for a purpose of description, and shall not be construed to indicate or imply relative importance or imply the number of indicated technical features. Therefore, features defining the terms such as "first" "second", and "third" can explicitly or implicitly include at least one of the features. In descriptions of the present disclosure, unless otherwise explicitly and specifically defined, "a plurality of" or "multiple" means at least two, for example, two, three and the like. All directional indications (for example, up, down, left, right, front, back) in the embodiments of the present disclosure are only used for explaining relative position relationships, movement situations or the like between the various components in a specific posture (as shown in the accompanying drawings). "sub-metal layer" is to be understood as metal sub-layer. When the specific posture changes, the directional indications change accordingly. In the embodiments of present disclosure, the terms "include", "have", and any variant thereof are intended to cover a non-exclusive inclusion. For example, a process, a method, a system, a product, or a device including a series of operations or units is not limited to listed operations or listed units. Instead, an operation or a unit which is not listed may be further included, or other operations or components inherent to the process, the method, the product, or the device.

The term "Embodiment" mentioned in the specification means which particular features, structures, or characteristics described with reference to the embodiments may be included in at least one embodiment of the present disclosure. The term appearing at different positions of the specification may not refer to the same embodiment or an independent or an alternative embodiment mutually exclusive with other embodiments. A person skilled in the art explicitly or implicitly understands which the embodiments described in the specification may be combined with other embodiments.

The present disclosure is described in detail in the following with reference to the accompanying drawings and embodiments.

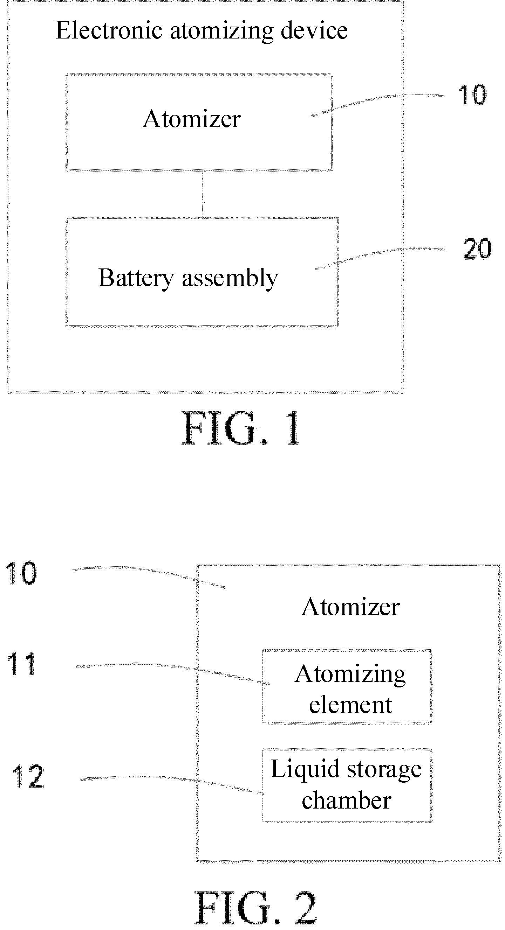

FIG. 1 is a schematic structural diagram of an electronic atomizing device according to some embodiments of the present disclosure. In the present embodiment, an electronic atomizing device is provided. The electronic atomizing device may be configured to atomize an aerosol-generating substrate substance. The electronic atomizing device may include an atomizer 10 and a battery assembly 20 electrically connected to atomizer 10.The atomizer 10 is configured to store the aerosol-generating substance and atomize the aerosol-generating substance to generate the aerosol which may be inhaled by a user. The atomizer 10 may be specifically applied in different fields. For example, the atomizer 10 may be applied to a medical treatment device, or an electronic aerosolization device. In a specific embodiment, the atomizer 10 may be applied in the electronic aerosolization device and configured to atomize a to-be-atomized substrate and generate the aerosol for an inhaler to inhale. All the following embodiments take this case as an example. Certainly, in other embodiments, the atomizer 10 may be applied to a medical device configured for treating a disease of an upper and lower respiratory system to atomize a medical drug.

For a specific structure and a specific function of the atomizer 10, reference may be made to the specific structure and the specific function of the atomizer 10 in any of the following embodiments, and the same or similar technical effects may be achieved. Details are not repeated herein.

The battery assembly 20 may include a battery (not shown in the figure) and a controller (not shown in the figure). The battery is configured to supply power to the atomizer 10, such that the atomizer 10 may atomize the aerosol-generating substance to generate an aerosol. The controller is configured to control the atomizer 10 operating.

The atomizer 10 and the battery assembly 20 may be integrally arranged or detachably connected, which may be designed according to specific needs.

As shown in FIG. 2, FIG. 2 is a schematic structural diagram of an atomizer according to some embodiments of the present disclosure. In the present embodiment, the atomizer 10 is provided. The atomizer 10 may include an atomizing element 11 and a liquid storage chamber 12. The liquid storage chamber 12 may be configured to store the aerosol-generating substance. The atomizing element 11 may be configured to heat and atomize the aerosol-generating substance in the liquid storage chamber 12. For a specific structure and a specific function of the atomizing element 11, reference may be made to the specific structure and the specific function of the atomizing element 11 in any of the following embodiments, and the same or similar technical effects may be achieved. Details are not repeated herein.

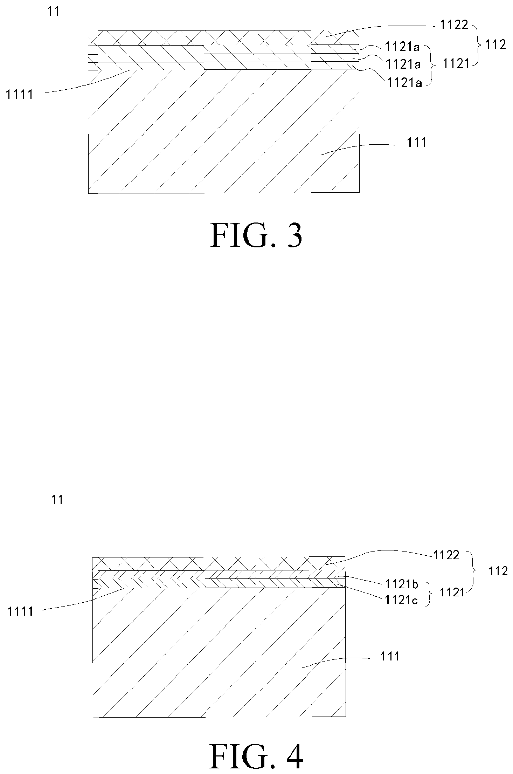

FIG. 3 is a schematic structural diagram of an atomizing element 11 according to a first embodiment of the present disclosure. In the present embodiment, an atomizing element 11 is provided. The atomizing element 11 may include a substrate 111 and a heating film 112. The substrate 111 may include an atomizing surface 1111. The heating film 112 is arranged on the atomizing surface 1111, such the heating film 112 may heat the aerosol-generating substance close to the atomizing surface 1111 to generate the aerosol in response to being energized.

Specifically, the substrate 111 may be a porous material, such as a porous ceramic, a porous glass, a porous plastic, or a porous metal. In the present embodiment, a material of the substrate 111 may be the porous ceramic substrate. The porous ceramic has pores and has a function of conducting a liquid and a function of storing a liquid, such that the aerosol-generating substance may permeate into the atomizing surface 1111 in the substrate 111 to be heated and atomized. The porous ceramic has stable chemical properties and does not chemically react with the aerosol-generating substance. The porous ceramic is high-temperature resistant, and does not deform due to an excessive heating temperature. The porous ceramic is an insulator which is not electrically connected to the heating film 112 on the porous ceramic, such that a possibility of a short circuit may be reduced. In addition, the porous ceramic is easy to manufacture and low in cost. In an embodiment, a liquid storage groove may be defined in a surface of the substrate 111 opposite to the atomizing surface 1111(not shown in the figure). The aerosol-generating substance in the liquid storage chamber 12 may first enter into the liquid storage groove, and then permeate into the atomizing surface 1111.

In some embodiments, a porosity of the porous ceramic may be in a range of 30% to 70%. The porosity is a ratio of a total volume of tiny gaps in a porous medium to a total volume of the porous medium. A value of the porosity may be adjusted according to a composition of the aerosol-generating substance. For example, when a viscosity of the aerosol-generating substance is relatively high, a greater porosity may be selected to ensure a liquid-conducting effect.

In some embodiments, the porosity of the porous ceramic may be in a range of 50% to 60%. With the porosity of the porous ceramic being the range of 50% to 60%. The porous ceramic may have a relatively high liquid-conducting efficiency, such that a possibility of dry burning caused by poor circulation of the aerosol-generating substance may be reduced, so as to improve the atomizing effect. Further, a possibility of a liquid leakage due to the porous ceramic conducting the liquid excessively fast and the liquid being difficult to be blocked may be reduced.

In some embodiments, a modification layer may be further arranged on the atomizing surface 1111 of a porous substrate 111. Especially when the material of the porous substrate 111 is the porous ceramic, a surface smoothness of the porous ceramic is extremely low, resulting in a difficulty of depositing a continuous and compact porous structure on a surface of the metal heating layer 1121. The modification layer may improve a surface flatness of the atomizing surface 1111 of the porous substrate 111, such that the continuous and compact porous structure may be deposited on the surface of the metal heating layer 1121. A thickness of the modification layer may be in a range of 50 µm to 300 µm. It may be understood that a modification effect is poor in response to the thickness of the modification layer being excessively small. An excessively thick modification layer may cover a large number of pores of the porous substrate, such that a thermal conductivity may be reduced.

Further, the modification layer may include a composition in parts by mass of: 56 to 67.5 parts of silicon dioxide, 12 to 18 parts of aluminum oxide, and 2.8 to 5.5 parts of lithium oxide. In some embodiments, the modification layer may further include 1.8 to 2.8 parts of phosphorus pentoxide, 0.5 to 2.0 parts of calcium oxide, 0.15 to 1.5 parts of magnesium oxide, and 2.5 to 5.25 parts of barium oxide. Further, in some embodiments, the modification layer may further include a following compositions in parts by mass: at least one of 0.1 to 5 parts of zirconium oxide and 0.3 to 0.45 parts of zinc oxide.

The heating film 112 may include an inorganic protection layer 1122 and the metal heating layer 1121. The metal heating layer 1121 is configured to heat in response to being energized, so as to heat and atomize the aerosol-generating substance. The metal heating layer 1121 is arranged on the substrate 111. In the present embodiment, the metal heating layer 1121 is arranged on the atomizing surface 1111 of the substrate 111. The metal heating layer 1121 includes multiple stacked sub-metal layers 1121a. The number of the sub-metal layers 1121a e may be two or more. In the present embodiment, the number of the sub-metal layers 1121a is three. In the metal heating layer 1121, any two adjacent sub-metal layers 1121a have different compositions. That is, the metal heating layer 1121 may include at least two sequentially stacking sub-metal layers 1121a having different compositions. Two adjacent sub-metal layers 1121a have different compositions. For example, when the metal heating layer 1121 includes a sub-metal layer A and a sub-metal layer B stacked together and having different compositions, and the number of the sub-metal layers 1121a is four, a structure of the metal heating layer 1121 may be A-B-A-B or B-A-B-A. When the metal heating layer 1121 includes the sub-metal layer A, the sub-metal layer B, and a sub-metal layer C stacked together and having different compositions, and the number of the sub-metal layers 1121a is five, the structure of the metal heating layer 1121 may be A-B-C-B-A, or A-B-C-A-B. Transition may be performed between the sub-metal layers 1121a having different compositions, and some sub-metal layers 1121a may be configured as a transition to reduce a residual stress inside the metal heating layer 1121, such that a continuity of a film layer of the metal heating layer 1121 may be improved and cracks in a surface of the heating film 112 may be reduced. In this way, adhesion of the heating film 112 may be effectively improved, and the stability and the life of the atomizing element 11 may be improved.

Further, the heating film 112 is a porous film. A porous structure on the heating film 112 may cause a liquid aerosol-generating substance to permeate into the heating film 112 or a surface of the atomizing surface 1111 more efficiently, such that the liquid-conducting efficiency and the thermal conductivity of the heating film 112 may be improved, and the atomizing effect of the atomizing element 11 may be improved. It may be understood that the inorganic protection layer 1122 and the metal heating layer 1121 of the heating film 112 may also be porous films. Specifically, in some embodiments, the inorganic protection layer 1122 and the metal heating layer 1121 are continuous porous structures. The metal heating layer 1121 may be the metal film deposited on the porous substrate 111 by means of a physical vapor deposition (PVD) or a chemical vapor deposition (CVD). In a film forming process, porous structures corresponding to the pores in the porous substrate 111 may be formed, and a continuous and compact metal film may include a solid portion between the pores. Therefore, the continuous porous structures may be formed in a preparation process. A parameter such as a pore size and a porosity of the heating film 112 may be adjusted by controlling a parameter such as a porosity and pore size of the porous substrate 111 and a thickness of the metal film. Similarly, a porous structure of the inorganic protection layer 1122 may also be formed in the preparation process. In the sintering process of a raw material coating of the inorganic protection layer 1122, due to the fluidity of the raw material coating, a part of the raw material coating enters into the pores of the porous substrate 111, and the other part of the raw material coating is continuously and compactly covered on the metal heating layer 1121 and form the continuous porous structure.

The inorganic protection layer 1122 is arranged on a surface of the metal heating layer 1121 away from the substrate 111. The inorganic protection layer 1122 is configured to protect the metal heating layer 1121. Specifically, the inorganic protection layer 1122 is arranged on a surface of the outermost sub-metal layer 1121a of the metal heating layer 1121 away from the substrate 111. The heating film 112 only arranged with the metal heating layer 1121 is prone to being corroded by the aerosol-generating substance or an atomized aerosol, causing a failure of the metal heating layer 1121, and resulting in a poorer stability and a shorter life of the atomizing element 11. The inventor finds that a resistance value of the heating film 112 may be reduced by adding the inorganic protection layer 1122. In addition, an increasing magnitude of the resistance value of the heating film 112 may also be significantly reduced during use. It is proved that a stability of the heating film 112 during use is improved, such that a possibility of a failure of the heating film 112 may be effectively reduced when the oil supply is insufficient, and the life of the atomizing element 11 may be improved. For an implementation of the foregoing effects, after an analysis, the inventor believes that when the inorganic protection layer 1122 is arranged on the surface of the metal heating layer 1121, the inorganic protection layer 1122 may reduce a possibility of the aerosol-generating substance or the atomized aerosol contacting with the metal heating layer 1121, such that a corrosion on the metal heating layer 1121 by the aerosol-generating substance or the atomized aerosol may be greatly reduced, and the life of the atomizing element 11 may be prolonged.

In the present disclosure, the internal stress of the metal heating layer 1121 may be reduced by arranging multiple stacked sub-metal layers 1121a as the metal heating layer 1121. On the one hand, a continuity of the film layer of the metal heating layer 1121 may be improved, and an occurrence of the cracks may be reduced. On the other hand, a risk of a crack failure generated in the heating film 112 under a thermal shock may be reduced. In addition, the inorganic protection layer 1122 is arranged on the surface of the metal heating layer 1121 away from the substrate 111 and protects the metal heating layer 1121, so that a corrosion resistance of the heating film 112 may be further improved, and a life of the heating film 112 may be prolonged. Compared with a solution in which the precious metal is used as a protection layer, the heating film 112 may include no precious metal material or a usage amount of the precious metal material in the heating film 112 may be reduced, so that an overall cost is low, and a cost of the atomizing element 11 may be reduced. In addition, a difficulty of an occurrence of over-burning and an agglomeration under a condition of an insufficient oil supply or the thermal shock may be improved, so that the metal heating layer 1121 is not prone to the failure, the stability of the atomizing element 11 may be effectively improved, and the life of the atomizing element 11 may be prolonged.

A material of the inorganic protection layer 1122 may be selected according to actual needs. A sintering temperature of the material of the inorganic protection layer 1122 may be usually below 1000°C. For example, the material of the inorganic protection layer 1122 may be a ceramic material or a glass material. In some embodiments, when the inorganic protection layer 1122 mainly includes a ceramic material, the inorganic protection layer 1122 may mainly include at least one component of Al₂O₃, SiO₂, MgO, BaO, CaO, ZrO₂ or ZnO. In a sintering process, above components may be presented in the inorganic protection layer 1122 in a form of corresponding silicates such as Ca₂SiO₃, Mg₂SiO₃, or other compound compositions. In some embodiments, when the inorganic protection layer 1122 mainly includes a glass material, the inorganic protection layer 1122 may mainly include at least one of Na₂SiO₃, CaSiO₃, SiO₂, Li₂O or Na₂O·CaO·6SiO₂.

In an embodiment, to ensure a continuity of a liquid supply and reduce the possibility of the dry burning, the heating film 112 may be required to have a particular wettability. Based on specific atomizing substrates having different types, the heating film 112 may be substantially divided into a lipophilic heating film, a hydrophilic heating film, and a hydrophilic and lipophilic heating film. In the present embodiment, a surface feature of the heating film 112 are substantially determined by the inorganic protection layer 1122. Therefore, the inorganic protection layer 1122 has lipophilicity and/or hydrophilicity. For the lipophilicity and/or the hydrophilicity, the raw material may be modified by impregnating with different solvents or additives or changing a process condition to prepare a lipophilic and/or hydrophilic inorganic protection layer 1122. For example, a mixture of hydrogen-containing silicone oil, ethanol, and sodium acetate may be selected as a modifying solution. A ceramic raw material may be impregnated at a room temperature and be heated and dried at about 100°C to prepare a lipophilic ceramic raw material. The inventor further finds through a research that when the inorganic protection layer 1122 is prepared by sintering the raw material of the inorganic protection layer 1122 at 400°C to 650°C, the inorganic protection layer 1122 shows a good wettability to a liquid atomized substrate, i.e., the lipophilicity and the hydrophilicity. Compositions of the liquid atomized substrate may include propylene glycol (PG), glycerol (vegetable glycerin/VG), essence, and the like.

A thickness of the inorganic protection layer 1122 may be in a range of 0.1 µm to 5 µm. For example, the thickness of the inorganic protection layer 1122 may be 0.1 µm, 1 µm, 2 µm, 3 µm, 4 µm, 5 µm, or the like. An excessively thin inorganic protection layer 1122 may provide an inadequate protection for the metal heating layer 1121. An excessively thick inorganic protection layer 1122 may cover the substrate 111 and a porous structure of the metal heating layer 1121 in the preparation process, making it difficult for the heating film 112 to form a continuous porous grid structure and reducing the liquid-conducting efficiency and the thermal conductivity of the heating film 112. A range of the thickness of the inorganic protection layer 1122 is set within a range from 0.1 µm to 5 µm, such that an effective protection of the inorganic protection layer 1122 to the metal heating layer 1121 may be achieved, and the continuous porous grid structure may be formed in the heating film 112.The liquid-conducting efficiency and the thermal conductivity of the heating film 112 may be better.

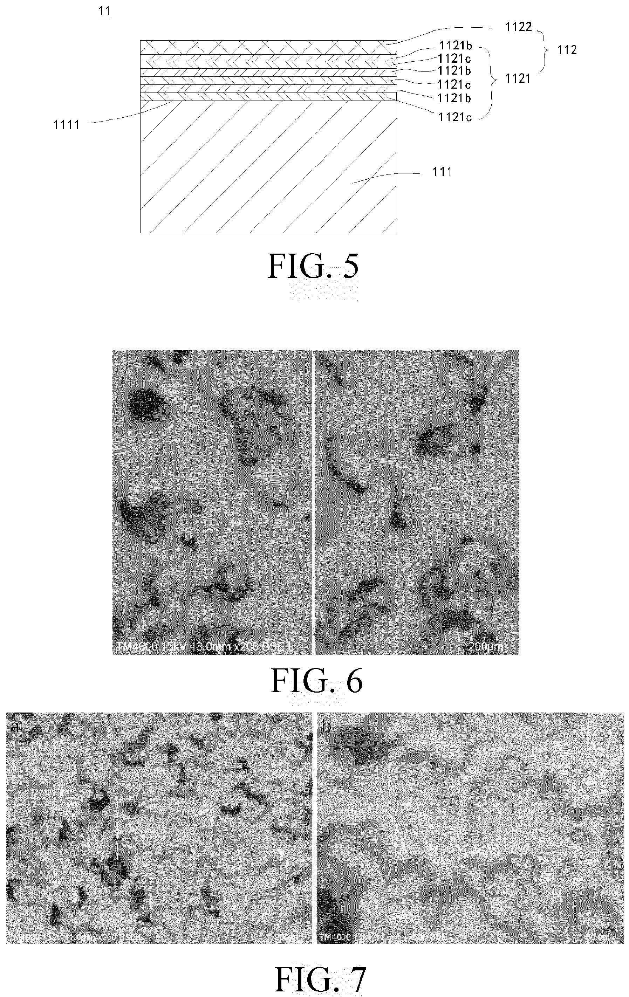

FIG. 4 is a schematic structural diagram of the atomizing element 11 according to a second embodiment of the present disclosure. FIG. 5 is a schematic structural diagram of the atomizing element 11 according to a third embodiment of present disclosure.

The metal heating layer 1121 may include a first sub-metal layer 1121b and a second sub-metal layer 1121c. The first sub-metal layer 1121b serves as a heating layer of the metal heating layer 1121 to generate heat. A material of the first sub-metal layer 1121b may include a metal or an alloy material with a good continuity, a good oxidation resistance, and a corrosion resistance, e.g., a NiCr alloy or a 316L stainless steel. The NiCr alloy has a high resistivity and a good thermal stability. The 316L stainless steel has an excellent corrosion resistance and a high-temperature resistance strength due to an addition of a Mo element.

The second sub-metal layer 1121c may be arranged between the first sub-metal layer 1121b at the lowermost and the substrate 111 (as shown in FIG. 4), and contacting with the atomizing surface 1111 to serve as a transition layer between the substrate 111 and the first sub-metal layer 1121b at the most. Or when there are a plurality of first sub-metal layers 1121b, the second sub-metal layer 1121c may be arranged between adjacent first sub-metal layers 1121b (as shown in FIG. 5) as a transition layer between the plurality of first sub-metal layers 1121b. In an embodiment, the second sub-metal layer 1121c may be arranged between the substrate 111 and the first sub-metal layer 1121b at the lowermost and also arranged between the adjacent first sub-metal layers 1121b. A material of the second sub-metal layer 1121c may include a titanium zirconium (TiZr) alloy.

FIG. 6 is a scanning electron micrograph of a heating film 112 prepared by only arranging the first sub-metal layer 1121b (including the NiCr alloy or the 316L stainless steel) on the substrate 111. It can be seen from FIG. 6, only the heating film 112 of the first sub-metal layer 1121b is arranged. Since both a NiCr film and a 316L stainless steel film have good continuities, a great residual stress may exist on a surface of the heating film. After sintering, a large number of cracks may exist on the surface of the heating film 112, and an adhesion of the film layer is poor. As a result, the film layer severely falls off, so that the stability and the life of the heating film 112 may be severely affected.

The second sub-metal layer 1121c is arranged as the transition layer between the plurality of first sub-metal layers 1121b or the transition layer between the first sub-metal layer 1121b and the substrate 111, so that a residual stress on a surface of the first sub-metal layer 1121b may be effectively reduced and the cracks on the surface of the heating film 112 may be reduced. In this way, a situation in which the film layer falls off may be effectively improved, and the stability and the life of the atomizing element 11 may be improved.

As shown in FIG. 4 and FIG. 5, in an embodiment, the first sub-metal layer 1121b and the second sub-metal layer 1121c are alternately distributed between the atomizing surface 1111 and the inorganic protection layer 1122. The second sub-metal layer 1121c may be in direct contact with the atomizing surface 1111, or the first sub-metal layer 1121b may be in direct contact with the atomizing surface 1111. The number of the first sub-metal layers 1121b and the number of second sub-metal layers 1121c may be the same or may be different.

In some embodiments, the number of the first sub-metal layers 1121b and the number of the second sub-metal layers 1121c are the same, the second sub-metal layer 1121c is in direct contact with the atomizing surface 1111, and the outermost sub-metal layer is the first sub-metal layer 1121b. For example, as shown in FIG. 4, in the embodiment according to FIG. 4, both the number of the first sub-metal layer 1121b and the number of the second sub-metal layer 1121c are one, and the second sub-metal layer 1121c is in direct contact with the atomizing surface 1111. As shown in FIG. 5, both the number of the first sub-metal layers 1121b and the number of the second sub-metal layers 1121c are three, and one second sub-metal layer 1121c is in direct contact with the atomizing surface 1111. The second sub-metal layer 1121c is arranged attaching to the surface of the substrate 111, so that a situation in which the surface of the heating film 112 has many cracks and the heating film 112 tends to fall off may be effectively improved. In a layered structure in which the first sub-metal layer 1121b and the second sub-metal layer 1121c are alternately arranged, the number of the first sub-metal layers 1121b and the number of the second sub-metal layers 1121c are the same. The second sub-metal layer 1121c is in direct contact with the atomizing surface 1111, and the outermost sub-metal layer is the first sub-metal layer 1121b. The first sub-metal layer 1121b is on the uppermost layer, so that a possibility of the failure of the metal layer 1121 may be reduced when the oil supply is insufficient or liquid oil is semi-dry.

Further, in the structure in which the first sub-metal layer 1121b and the second sub-metal layer 1121c are alternately arranged, a total number of layers may be two to ten layers. For example, as shown in FIG. 4, in the second embodiment, the total number of the layers are two. Both the number of the first sub-metal layer 1121b and the number of the second sub-metal layer 1121c are one, and the second sub-metal layer 1121c is arranged between the first sub-metal layer 1121b and the substrate 111.

The number of the first sub-metal layers 1121b and the number of the second sub-metal layers 1121c may also be at least two. As shown in FIG. 5, in the third embodiment, the total number of the layers in the layered structure in which the first sub-metal layer 1121b and the second sub-metal layer 1121c are alternately arranged may be six. The number of the first sub-metal layers 1121b is three, and the number of the second sub-metal layers 1121c is also three. Three first sub-metal layers 1121b and three second sub-metal layers 1121c are alternately stacked. In some embodiments, materials of the three second sub-metal layers 1121c are all the TiZr alloy, and materials of the first sub-metal layers 1121b are all the 316L or the NiCr alloy. A layered structure including the second sub-metal layers 1121c and the first sub-metal layers 1121b may be TiZr-316L-TiZr-316L-TiZr-316L or TiZr-NiCr-TiZr-NiCr-TiZr-NiCr alloy. When the number of the layers in the layered structure in which the first sub-metal layer 1121b and the second sub-metal layer 1121c are alternately arranged is less, under a condition that a total resistance of the heating film 112 is kept unchanged, a total thickness of the heating film 112 may remain unchanged, and a thickness of a single metal layer 1121 may be increased. Stresses accumulated by metal particles in a single layer deposition process may be more, resulting in more cracks of the heating film 112. When the total number of layers of the layered structure in which the first sub-metal layer 1121b and the second sub-metal layer 1121c are alternately arranged are six, the thickness of the single metal layer 1121 may be relatively moderate, the heating film 112 formed has fewer cracks and has a stronger thermal shock resistance, and a service life of the atomizing element 11 may be longer.

It may be understood that when both the number of the first sub-metal layers 1121b and the number of the second sub-metal layers 1121c are at least two, a total thickness of the first sub-metal layers 1121b may be in a range of 1 µm to 20 µm, e.g., 1 µm, 5 µm, 10 µm, 15 µm, 20 µm, or the like. A total thickness of the second sub-metal layers 1121c may be in a range of 0.5 µm to 5 µm, e.g., 0.5 µm, 1 µm, 2 µm, 3 µm, 4 µm, or 5 µm. The total thickness is a sum of thicknesses of all the same sub-metal layers. A thickness of a single sub-metal layer may be adjusted according to the actual needs. The thickness of the single sub-metal layer and the total thickness are usually adjusted based on a requirement of a resistance value of the heating film 112. For example, in an embodiment, the number of the layered structure of the metal heating layer 1121 may be six. The number of the first sub-metal layers 1121b is three and the number of the second sub-metal layers 1121c is three. The total thickness of the first sub-metal layers 1121b may be 6 µm, and the total thickness of the second sub-metal layers 1121c may be 3 µm. The thickness of the single sub-metal layer may be the same or different. In the present embodiment, the thickness of the single sub-metal layer may be the same. Thicknesses of single first sub-metal layers 1121b are all 2 µm, and thicknesses of single second sub-metal layers 1121c are all 1 µm. In some embodiments, the thicknesses of the single sub-metal layers may be partially or completely the same. For example, in a direction from the substrate 111 to the inorganic protection layer 1122, the thicknesses of the second sub-metal layers 1121c are sequentially 0.5 µm, 2 µm, and 0.5 µm, and the total thickness is 3 µm. The thicknesses of the first sub-metal layers 1121b are sequentially 1 µm, 2 µm, and 3 µm, and the total thickness is 6 µm. It may be understood that, in some embodiments, the metal heating layer 1121 may be in direct contact with the substrate 111 through the second sub-metal layer 1121c. The second sub-metal layer 1121c serves as a transition layer, and consequently the thickness of the second sub-metal layer 1121c being in direct contact with the inorganic protection layer 1122 or the porous substrate 111 may be less than thicknesses of other second sub-metal layers 1121c, so that the continuity and a compactness of the heating film 112 may be improved and the cracks may be reduced. In this way, a possibility of a failure under the thermal shock may be reduced, and the service life of the atomizing element 11 may be prolonged. FIG. 7 is a scanning electron micrograph according to an embodiment of the present disclosure. In FIG. 7a, the surface of the heating film 112 is continuous and compact and has a distribution of a specific amount of pores. The heating film 112 is presented to be a continuous porous structure as a whole. FIG. 7b is a partial enlarged view of FIG. 7a, and shows a contrast example of the heating film 112 relative to FIG. 6. The heating film 112 is continuous and compact, has no cracks, and completely covers a non-porous region of the substrate.

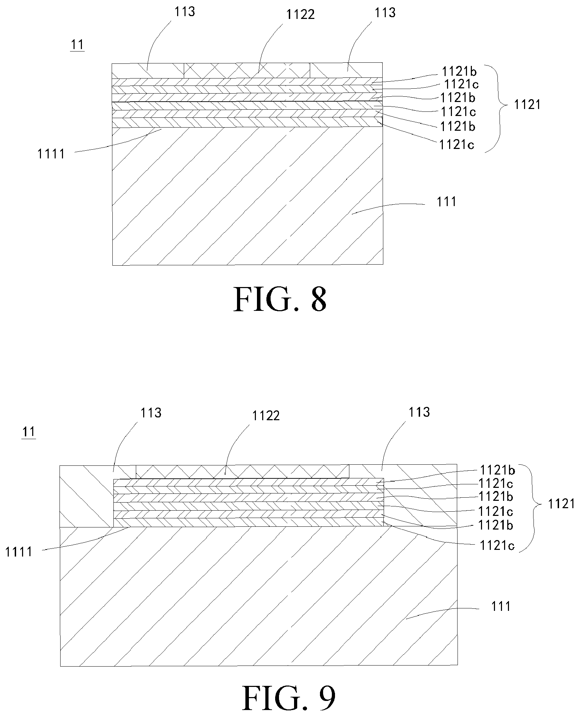

As shown in FIG. 8 and FIG. 9, FIG. 8 is a schematic structural diagram of the atomizing element 11 according to a fourth embodiment of the present disclosure, and FIG. 9 is aschematic structural diagram of the atomizing element 11 according to a fifth embodiment of the present disclosure.

The atomizing element 11 may further include two electrodes 113. One of the two electrodes 113 is arranged on a first side of the metal heating layer 1121, the other one of the two electrodes 113 is arranged on a second side of the metal heating layer opposite to the first side. The two electrodes 113 are electrically connected to the metal heating layer 1121. The inorganic protection layer 1122 covers a portion of the uppermost sub-metal layer 1121a located between the two electrodes 113. The one of the two electrodes 113 is electrically connected to a positive lead wire and the other one of the two electrodes 113 is electrically connected to a negative lead wire, so that the metal heating layer 1121 between the two electrodes 113 may conduct a current and generate heat. The two electrodes 113 may be partially or completely arranged on a side of the uppermost sub-metal layer 1121a away from the substrate 111. In the fourth embodiment, as shown in FIG. 8, the two electrodes 113 may be completely arranged on a part of a surface of the uppermost sub-metal layer 1121a away from the substrate 111. The one of the two electrodes 113 is arranged on a first end of the uppermost sub-metal layer 1121a, and the other one of the two electrodes 113 is arranged on a side opposite to the first side of the uppermost sub-metal layer 1121a at an interval. The inorganic protection layer 1122 covers the other part of the surface of the uppermost sub-metal layer 1121a away from the substrate 111. In the fifth embodiment, as shown in FIG. 9, the metal heating layer 1121 is arranged on a part of the atomizing surface 1111 of the substrate 111, and parts of the two electrodes 113 are arranged on the uppermost sub-metal layer 1121a. The other parts of the two electrodes 113 are arranged on the other part of the atomizing surface 1111 of the substrate 111. The inorganic protection layer 1122 covers the portion of the uppermost sub-metal layer 1121a between the two electrodes 113. Materials of the two electrodes 113 may be selected from metal materials having good conductivities such as gold, and silver, etc. In a consideration of both the cost and a preparing ease, silver is preferred.

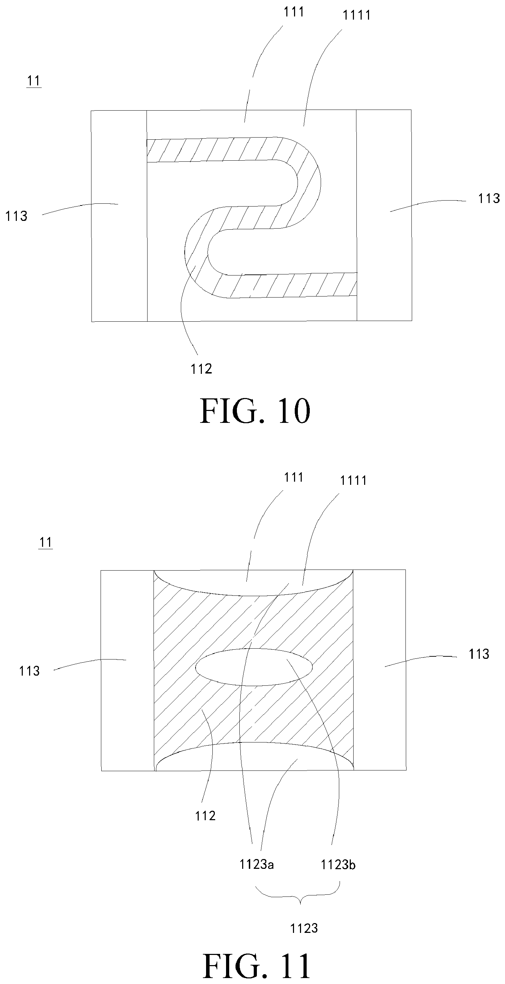

As shown in FIG. 10 and FIG. 11, FIG. 10 is a top view of the atomizing element 11 according to a sixth embodiment of the present disclosure. FIG. 11 is a top view of the atomizing element 11 according to a seventh embodiment of the present disclosure. A shaded part is the heating film 112 of the atomizing element 11 or the metal heating layer 1121. In the present embodiment, a shape of the heating film 112 or a shape of the metal heating layer 1121 may be a preset pattern, which may be a regular pattern such as substantially an S shape, a circle, an ellipse, a semicircle, or a ring. For example, in the embodiment according to FIG. 10, the shape of the heating film 112 is substantially the S shape. The one of the two electrodes 113 is connected to one end of an S-shaped heating film 112, and the other one of the two electrodes 113 is connected to the other side of the S-shaped heating film 112.

The shape of the heating film 112 may also be an irregular pattern. For example, in the embodiment according to FIG. 11, the shape of the heating film 112 is the irregular pattern. In some embodiments, the heating film 112 may be the regular pattern, and one or more hollows 1123 are defined on the regular pattern. For example, in the embodiment according to FIG. 11, the shape of the heating film 112 may be a substantially rectangle. The hollows 1123 which does not generate heat are defined in a rectangular heating film. The hollows 1123 includes first hollows 1123a and a second hollow 1123b. The number of first hollows 1123a is two. The one of the two electrodes 113 is arranged on a first side of the rectangle, and the other one of the two electrodes 113 is symmetrically arranged on a second side of the rectangle opposite to the first side. A first one of the first hollows 1123a is located on a third side of the rectangle, a second one of the first hollows 1123a is located on a fourth side of the rectangle opposite to the third side of the rectangle. The third side of the rectangle is substantially vertical to the first side of the rectangle. In some embodiments, shapes of the first hollows 1123a may be substantially fan-shaped. The second hollow 1123b is defined between the two first hollows 1123a and is located in a middle of the heating film 112. In some embodiments, a shape of the second hollow 1123b may be substantially an ellipse. In other embodiments, the shape of the heating film 112 may also be substantially a square, a triangle, the circle, a semicircle, or the like. Shapes of the first hollows 1123a and the second hollow 1123b may be substantially the circles, the triangles, the rectangles, the squares, or the like. The number of the first hollows 1123a may be one or more, and the number of the second hollows 1123b may be one or more. When the heating film 112 is not patterned, the greatest temperature for the aerosol-generating substance being atomized by the heating film 112 is between 210°C and 230°C, so that a generated aerosol is difficult to evaporate a fragrance, a stimulation sense is weaker, and a taste is poor. Through patterning the heating film 112, the heating film 112 may construct a better heating temperature gradient according to the shape of the heating film 112. In this way, a surface power density may be increased, and a high temperature region of the heating film 112 may be increased. The aerosol generated by an atomizing process performed by the heating film 112 may have a stronger sweetness and a stronger fragrance. The stimulation sense may be stronger, so that the taste of the aerosol may be effectively improved.

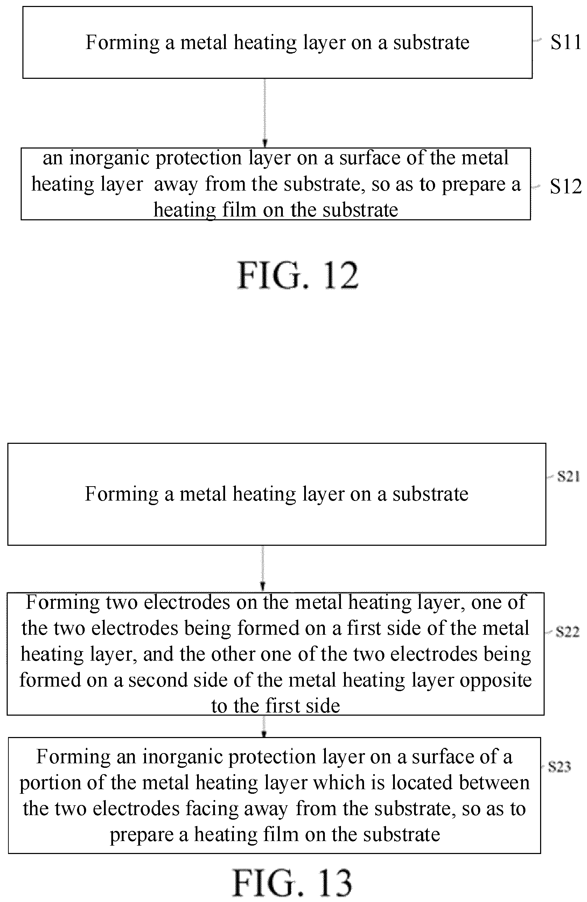

As shown in FIG. 12, FIG. 12 is a flowchart of a preparation method for the atomizing element according to the first embodiment of the present disclosure.

In the present embodiment, the preparation method for the atomizing element may include operations S11 and S12.

At block S11, forming a metal heating layer on a substrate.

The substrate 111 may be a porous ceramic substrate. The metal heating layer 1121 may include at least two sequentially stacked sub-metal layers 1121a. Any two adjacent sub-metal layers 1121a may have different compositions. That is, the metal heating layer 1121 may include the at least two sequentially stacked sub-metal layers 1121a having different compositions, and the two adjacent sub-metal layers 1121a have different compositions. Transitions may be performed between the sub-metal layers 1121a having different compositions. Some sub-metal layers 1121a may be configured as transitions to reduce a residual stress inside the metal heating layer 1121, so that the continuity of the film layer of the metal heating layer 1121 may be improved and cracks on a surface of the heating film 112 may be reduced. In this way, an adhesion of the heating film 112 may be effectively improved, and the stability and the service life of the atomizing element 11 may be improved.

In a specific implementation process, raw materials of the at least two sub-metal layers 1121a configured to form the metal heating layer 1121 may be separately made into various target materials. Then, a magnetron sputtering process is performed to sputter the various target materials on an atomizing surface 1111 of the substrate 111 multiple times and obtain the metal heating layer 1121. The metal heating layer 1121 is formed on a surface of the substrate 111 by the magnetron sputtering process. The metal heating layer 1121 has a high purity, a good adhesion, a uniform thickness of a film, and a good process repeatability.

At block S12, forming an inorganic protection layer on a surface of the metal heating layer away from the substrate, so as to prepare a heating film on the substrate.

Specifically, the inorganic protection layer 1122 is configured to protect the metal heating layer 1121. A material of the inorganic protection layer 1122 may be at least one of a ceramic material or a glass material. In some embodiments, when the material of the inorganic protection layer 1122 is the ceramic material, the inorganic protection layer 1122 may substantially include at least one component of Al₂O₃, SiO₂, Ca₂SiO₃, and Mg₂SiO₃. In some other embodiments, when the material of the inorganic protection layer 1122 includes a glass material, the inorganic protection layer 1122 may substantially include Na₂SiO₃, CaSiO₃, SiO₂, Li₂O, and Na₂O·CaO·6SiO₂, etc..

After a prepared filter material of the inorganic protection layer 1122 is dried, the inorganic protection layer 1122 is formed on the surface of the metal heating layer 1121 facing away from the substrate 111 by performing a spraying process or a magnetron sputtering process, such that the heating film 112 is prepared on the substrate 111. When the spraying process is adopted in the filter material of the inorganic protection layer 1122, the spraying process may be performed on the surface of the metal heating layer 1121 facing away from the substrate 111 in a way of a small amount each time and repeating multiple times. In an embodiment, an intermediate obtained after the spraying process is completed may be dried in an oven at 80°C for 15 min. The dried intermediate may be sintered at a temperature of 600°C, and then cooled so as to prepare the heating film 112.

In the preparation method for the atomizing element, an internal stress of the metal heating layer 1121 may be reduced by arranging multiple layers of sub-metal layers 1121a which are stacked as the metal heating layer 1121. On the one hand, the continuity of the film layer of the metal heating layer 1121 may be improved, and an occurrence of the cracks may be reduced. On the other hand, a risk of a crack failure generated in the heating film 112 under a thermal shock may be reduced. In addition, the inorganic protection layer 1122 is arranged on the surface of the metal heating layer 1121 away from the substrate 111 and configured to protect the metal heating layer 1121, so that a corrosion resistance of the heating film 112 may be further improved, and a service life of the heating film 112 may be prolonged. Compared with a solution in which a precious metal is used as a protection layer, the heating film 112 does not include a precious metal material or the usage of the precious metal material in the heating film 112 may be reduced, so that an overall cost may be low, and a cost of the atomizing element 11 may be reduced. In addition, the heating film 112 is not prone to over-burning and agglomerating under a condition of an insufficient oil supply or the thermal shock, so that the metal heating layer 1121 is not prone to have a failure, the stability of the atomizing element 11 may be effectively improved, and the service life of the atomizing element 11 may be prolonged.

FIG. 13 is a flowchart of a preparation method for the atomizing element according to the second embodiment of the present disclosure. In the present embodiment, the preparation method for the atomizing element may include the following operations S21-S23.

At block S21, forming a metal heating layer on a substrate.

Specifically, a specific implementation process of the block S21 is the same as or similar to the specific implementation process of the block S11 in foregoing embodiments corresponding to FIG. 11, and the same or similar technical effects may be achieved. For details, reference may be made to above descriptions, and the details are not repeated herein.

At block S22, forming two electrodes on the metal heating layer, one of the two electrodes being formed on a first side of the metal heating layer, and the other one of the two electrodes being formed on a second side of the metal heating layer opposite to the first side.

Specifically, the one of the two electrodes 113 may be formed on the first side of the metal heating layer 1121 and the other one of the two electrodes 113 may be formed on the second side of the metal heating layer 1121 opposite to the first side of the metal heating layer 1121 by a screen printing process. An intermediate obtained after being performed the screen printing process may be placed in the oven to be dried at 80°C for 30 min.

At block S23, forming an inorganic protection layer on a surface of a portion of the metal heating layer which is located between the two electrodes facing away from the substrate, so as to prepare a heating film on the substrate.

Specifically, after the two electrodes 113 are blocked by a mask plate, the inorganic protection layer 1122 may be formed by the spraying process or the magnetron sputtering process on the surface of the portion of the metal heating layer 1121 which is located between the two electrodes 113.

In the second embodiment, the one of the two electrodes 113 is formed on the first side of the metal heating layer 1121 and the other one of the two electrodes 113 is formed on the second side of the metal heating layer 1121 opposite to the first side of the metal heating layer 1121. The two electrodes 113 may be connected to external lead wires, so that the metal heating layer 1121 between the two electrodes 113 may be energized to generate the heat after the two electrodes 113 are energized.

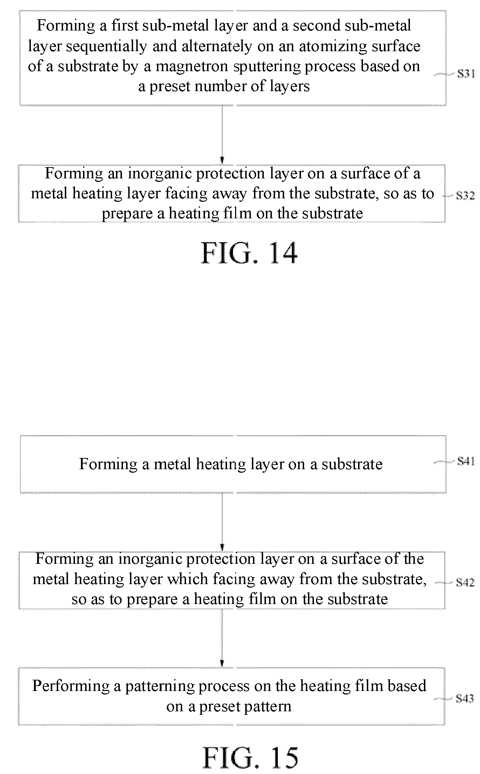

FIG. 14 is a flowchart of a preparation method for the atomizing element according to the third embodiment of the present disclosure. In the present embodiment, the preparation method for the atomizing element includes the following operations S31-S32.

At block S31, forming a first sub-metal layer and a second sub-metal layer sequentially and alternately on an atomizing surface of a substrate by a magnetron sputtering process based on a preset number of layers.

Specifically, the preset number of the layers may be a sum of the number of the first sub-metal layers 1121b and the number of the second sub-metal layers 1121c. The first sub-metal layer 1121b and the second sub-metal layer 1121c are alternately stacked between the atomizing surface 1111 and the inorganic protection layer 1122 based on the preset number of the layers. The second sub-metal layer 1121c may be configured to be in direct contact with the atomizing surface 1111, or the first sub-metal layer 1121b may be in direct contact with the atomizing surface 1111. The number of the second sub-metal layers 1121c may be the same with the number of the first sub-metal layers 1121b, or the number of the second sub-metal layers 1121c may be different from the number of the first sub-metal layers 1121b. In some embodiments, the number of the second sub-metal layers 1121c may be the same with the number of the first sub-metal layers 1121b, and the second sub-metal layers 1121c may be in direct contact with the atomizing surface 1111.

Specifically, a material of the first sub-metal layer 1121b may be a NiCr alloy or a 316L stainless steel. In an implementation, parameters of the magnetron sputtering process of the first sub-metal layer 1121b may be a sputtering power being 3500 W, a sputtering time being 80 min, a sputtering pressure being 0.5 Pa, and a sputtering temperature being in a range of a room temperature to 200°C. When a raw material of the first sub-metal layer 1121b is the NiCr, parameters of the magnetron sputtering process of the NiCr may be the sputtering power being 3500 W, the sputtering time being 60 min, the sputtering pressure being 0.5 Pa, and the sputtering temperature being 200°C. When the raw material of the first sub-metal layer 1121b is the 316L stainless steel, parameters of the magnetron sputtering process of the 316L stainless steel may be the sputtering power being 3000 W, the sputtering time being 80 min, the sputtering pressure being 0.5 Pa, and the sputtering temperature being 200°C.

A material of the second sub-metal layer 1121c may be a TiZr alloy. When the material of the second sub-metal layer 1121c is the TiZr alloy, in an implementation, parameters of the magnetron sputtering process for forming the second sub-metal layer 1121c may be a vacuum degree being 3.0*10⁻³ Pa, a sputtering power being 2500 W, a sputtering time being 30 min, a sputtering pressure being 0.5 Pa, a sputtering temperature being 200°C, and a sputtering particle size being in a range of 200 nm to 400 nm.

At block S32, forming an inorganic protection layer on a surface of a metal heating layer facing away from the substrate, so as to prepare a heating film on the substrate.

Specifically, a specific implementation process of the block S32 may be the same as or similar to the specific implementation process of the block S12 in the foregoing embodiments corresponding to FIG. 11. The same or similar technical effects may be achieved. For the details, the reference may be made to the above descriptions, and the details are not repeated herein.

In the third embodiment, the second sub-metal layer 1121c is arranged, so that a residual stress on a surface of the first sub-metal layer 1121b may be effectively reduced, cracks on a surface of the heating film 112 may be reduced, and the stability and the service life of the atomizing element 11 may be improved.

FIG. 15 is a flowchart of a preparation method for the atomizing element according to the fourth embodiment of the present disclosure. In the present embodiment, the preparation method for the atomizing element may include the following operations S41-S42.

At block S41, forming a metal heating layer on a substrate.

At block S42, forming an inorganic protection layer on a surface of the metal heating layer which facing away from the substrate, so as to prepare a heating film on the substrate.

Specifically, specific implementation processes of the block S41 and the block S42 are the same as or similar to specific implementation processes of the block S11 and the block S12 in the foregoing embodiments corresponding to FIG. 11. The same or similar technical effects may be achieved. For details, reference may be made to the above descriptions, and the details are not repeated herein.

At block S43, performing a patterning process on the heating film based on a preset pattern.

The preset pattern of the heating film 112 may be substantially an S shape, a circle, an ellipse, a semicircle, or a ring. As shown in FIG. 10, the preset pattern of the heating film 112 shown in FIG. 10 is a substantially S shape. The preset pattern of the heating film 112 may also be an irregular pattern, such as the irregular pattern shown in a shaded part in FIG. 11. Specifically, a hollow 1123 of the heating film 112 may be cut off through an etching process, so that a shape of the heating film 112 may be a preset pattern. For example, in the embodiments according to FIG. 11, first hollows 1123a and a second hollow 1123b of a substantially rectangular heating film 112 prepared in the block S42 may be removed through an etching process, so that an irregular heating film in FIG. 11 may be prepared.

In the fourth embodiment, the heating film 112 is patterned, a better heating temperature gradient may be constructed according to the shape of the heating film 112. A surface power density may be increased, and a high temperature region of the heating film 112 may be increased. An aerosol generated through an atomizing process performed by the heating film 112 may have a stronger sweetness and a stronger fragrance, and a strong stimulation sense, so that a taste of the aerosol may be effectively improved.

The foregoing descriptions are merely some embodiments of the present disclosure, and a protection scope of the present disclosure is defined by the appended claims.

## Claims

1. An atomizing element (11), comprising:
a substrate (111), comprising:
an atomizing surface (1111); and
a heating film (112), arranged on the atomizing surface (1111), capable of heating and atomizing an aerosol-generating substance on the atomizing surface (1111) in response to being energized, and comprising:
a metal heating layer (1121); and
an inorganic protection layer (1122), stacked with the metal heating layer (1121), arranged on the surface of the metal heating layer (1121) away from the substrate (111);
wherein the metal heating layer (1121) comprises at least two sequentially stacked metal sub-layers (1121a), and any two adjacent metal sub-layers (1121a) have different compositions.

2. The atomizing element (11) according to claim 1, wherein the substrate (111) is a porous substrate; or the metal heating layer (1121) and/or the inorganic protection layer (1122) are porous structures.

3. The atomizing element (11) according to claim 1, wherein the inorganic protection layer (1122) has lipophilicity and/or hydrophilicity.

4. The atomizing element (11) according to claim 1, wherein the metal heating layer (1121) comprises:
a first metal sub-layer (1121b); and
a second metal sub-layer (1121c), stacked with the first metal sub-layer (1121b);
wherein the material of the first metal sub-layer (1121b) comprises a NiCr alloy or a 316L stainless steel, and the material of the second metal sub-layer (1121c) comprises a TiZr alloy.

5. The atomizing element (11) according to claim 4, wherein the metal heating layer (1121) is in direct contact with, the substrate (111) through the second metal sub-layer (1121c).

6. The atomizing element (11) according to claim 4 or 5, wherein the number of the first metal sub-layer (1121b) is at least two, and the number of the second metal sub-layer (1121c) is at least two.

7. The atomizing element (11) according to claim 4, wherein the total thickness of the first metal sub-layer (1121b) is in the range of 1 µm to 20 µm, the total thickness of the second metal sub-layer (1121c) is in the range of 0.5 µm to 5 µm, and the thickness of the inorganic protection layer (1122) is in the range of 0.1 µm to 5 µm.

8. The atomizing element (11) according to claim 1, wherein the shape of the heating film (112) is an S shape, a circle, an ellipse, a semicircle, or a ring; or the shape of the metal heating layer (1121) is an S shape, a circle, an ellipse, a semicircle, or a ring.

9. The atomizing element (11) according to claim 1, further comprising two electrodes (113) electrically connected to the metal heating layer (1121), wherein one of the two electrodes (113) is arranged on a first side of the metal heating layer (1121), the other one of the two electrodes (113) is arranged on a second side of the metal heating layer (1121) opposite to the first side of the metal heating layer (1121); and the inorganic protection layer (1122) covers a portion of the metal heating layer (1121) located between the two electrodes (113).

10. The atomizing element (11) according to claim 9, wherein the heating film (112) is a rectangle, the one of the two electrodes (113) is arranged on a first side of the rectangle, and the other one of the two electrodes (113) is arranged on a second side of the rectangle opposite to the first side of the rectangle, and a hollow (1123) which does not generate heat is defined in the heating film (112).

11. The atomizing element (11) according to claim 10, wherein the hollow (1123) comprises:
first hollows (1123a), being fan-shaped, wherein a first one of the first hollows (1123a) is located on a third side of the rectangle, a second one of the first hollows (1123a) being located on a fourth side of the rectangle opposite to the third side of the rectangle, and the third side of the rectangle is vertical to the first side of the rectangle; and
a second hollow (1123b), located on the middle of the heating film (112), and the shape of the second hollow (1123b) being an ellipse or a circle.

12. The atomizing element (11) according to claim 2, wherein a modification layer is further arranged on the atomizing surface (1111) of the porous substrate, and the thickness of the modification layer is in the range of 50 µm to 300 µm.

13. The atomizing element (11) according to claim 12, wherein the modification layer comprises a composition in parts by mass of 56 to 67.5 parts of silicon dioxide, 12 to 18 parts of aluminum oxide, and 2.8 to 5.5 parts of lithium oxide.

14. An atomizer (10), **characterized by** comprising:
a liquid storage chamber (12), configured to store an aerosol-generating substance; and
an atomizing element (11) according to any one of claims 1 to 13.

15. An electronic atomizing device, **characterized by** comprising:
a battery assembly (20); and an atomizer (10) according to claim 14;
wherein the battery assembly (20) is configured to supply power to the atomizer (10).

## Patentansprüche

1. Ein Zerstäubungselement (11), umfassend:
ein Substrat (111), umfassend:
eine Zerstäubungsoberfläche (1111); und
eine Heizfolie (112), die auf der Zerstäubungsoberfläche (1111) angeordnet ist, geeignet ist, eine auf der Zerstäubungsoberfläche (1111) befindliche aerosolerzeugende Substanz als Reaktion auf eine Bestromung zu erhitzen und zu zerstäuben, und die umfasst:
eine metallische Heizschicht (1121); und
eine anorganische Schutzschicht (1122), die mit der metallischen Heizschicht (1121) gestapelt ist und auf der von dem Substrat (111) abgewandten Oberfläche der metallischen Heizschicht (1121) angeordnet ist;
wobei die metallische Heizschicht (1121) mindestens zwei sequenziell gestapelte metallische Unterschichten (1121a) umfasst, und wobei jeweils zwei benachbarte metallische Unterschichten (1121a) unterschiedliche Zusammensetzungen aufweisen.

2. Das Zerstäubungselement (11) gemäß Anspruch 1, wobei das Substrat (111) ein poröses Substrat ist; oder wobei die metallische Heizschicht (1121) und/oder die anorganische Schutzschicht (1122) poröse Strukturen sind.

3. Das Zerstäubungselement (11) gemäß Anspruch 1, wobei die anorganische Schutzschicht (1122) lipophile und/oder hydrophile Eigenschaften aufweist.

4. Das Zerstäubungselement (11) gemäß Anspruch 1, wobei die metallische Heizschicht (1121) umfasst:
eine erste metallische Unterschicht (1121b); und
eine zweite metallische Unterschicht (1121c), die mit der ersten metallischen Unterschicht (1121b) gestapelt ist;
wobei das Material der ersten metallischen Unterschicht (1121b) eine NiCr-Legierung oder einen Edelstahl 316L umfasst, und wobei das Material der zweiten metallischen Unterschicht (1121c) eine TiZr-Legierung umfasst.

5. Das Zerstäubungselement (11) gemäß Anspruch 4, wobei die metallische Heizschicht (1121) über die zweite metallische Unterschicht (1121c) in direktem Kontakt mit dem Substrat (111) steht.

6. Das Zerstäubungselement (11) gemäß Anspruch 4 oder 5, wobei die Anzahl der ersten metallischen Unterschichten (1121b) mindestens zwei beträgt, und wobei die Anzahl der zweiten metallischen Unterschichten (1121c) mindestens zwei beträgt.

7. Das Zerstäubungselement (11) gemäß Anspruch 4, wobei die Gesamtdicke der ersten metallischen Unterschichten (1121b) im Bereich von 1 µm bis 20 µm liegt, die Gesamtdicke der zweiten metallischen Unterschichten (1121c) im Bereich von 0,5 µm bis 5 µm liegt, und die Dicke der anorganischen Schutzschicht (1122) im Bereich von 0,1 µm bis 5 µm liegt.

8. Das Zerstäubungselement (11) gemäß Anspruch 1, wobei die Form der Heizfolie (112) eine S-Form, ein Kreis, eine Ellipse, ein Halbkreis oder ein Ring ist; oder wobei die Form der metallischen Heizschicht (1121) eine S-Form, ein Kreis, eine Ellipse, ein Halbkreis oder ein Ring ist.

9. Das Zerstäubungselement (11) gemäß Anspruch 1, die weiter zwei Elektroden (113) umfasst, die elektrisch mit der metallischen Heizschicht (1121) verbunden sind, wobei eine der beiden Elektroden (113) an einer ersten Seite der metallischen Heizschicht (1121) angeordnet ist, die andere der beiden Elektroden (113) an einer zweiten Seite der metallischen Heizschicht (1121) angeordnet ist, die der ersten Seite der metallischen Heizschicht (1121) gegenüberliegt; und wobei die anorganische Schutzschicht (1122) einen Abschnitt der metallischen Heizschicht (1121) abdeckt, der zwischen den beiden Elektroden (113) liegt.

10. Das Zerstäubungselement (11) gemäß Anspruch 9, wobei die Heizfolie (112) rechteckig ist, eine der beiden Elektroden (113) an einer ersten Seite des Rechtecks angeordnet ist, die andere der beiden Elektroden (113) an einer zweiten Seite des Rechtecks, die der ersten Seite des Rechtecks gegenüberliegt, angeordnet ist, und wobei in der Heizfolie (112) eine Vertiefung (1123) vorgesehen ist, die keine Wärme erzeugt.

11. Das Zerstäubungselement (11) gemäß Anspruch 10, wobei die Vertiefung (1123) umfasst:
eine Mehrzahl erster Vertiefungen (1123a), die fächerförmig sind, wobei eine der ersten Vertiefungen (1123a) an einer dritten Seite des Rechtecks angeordnet ist, eine zweite der ersten Vertiefungen (1123a) an einer vierten Seite des Rechtecks, die der dritten Seite des Rechtecks gegenüberliegt, angeordnet ist, und wobei die dritte Seite des Rechtecks senkrecht zur ersten Seite des Rechtecks steht; und
eine zweite Vertiefung (1123b), die in der Mitte der Heizfolie (112) angeordnet ist, und wobei die Form der zweiten Vertiefung (1123b) eine Ellipse oder ein Kreis ist.

12. Das Zerstäubungselement (11) gemäß Anspruch 2, wobei eine Modifikationsschicht zusätzlich auf der Zerstäubungsoberfläche (1111) des porösen Substrats angeordnet ist, und wobei die Dicke der Modifikationsschicht im Bereich von 50 µm bis 300 µm liegt.

13. Das Zerstäubungselement (11) gemäß Anspruch 12, wobei die Modifikationsschicht eine Zusammensetzung in Massenanteilen von 56 bis 67,5 Teilen Siliziumdioxid, 12 bis 18 Teilen Aluminiumoxid und 2,8 bis 5,5 Teilen Lithiumoxid umfasst.

14. Ein Zerstäuber (10), **gekennzeichnet dadurch, dass** er umfasst:
eine Flüssigkeitsspeicherkammer (12), die konfiguriert ist, eine aerosolerzeugende Substanz zu speichern; und
ein Zerstäubungselement (11) gemäß einem der Ansprüche 1 bis 13.

15. Eine elektronische Zerstäubungsvorrichtung, **gekennzeichnet dadurch, dass** sie umfasst:
eine Batterieanordnung (20); und einen Zerstäuber (10) gemäß Anspruch 14;
wobei die Batterieanordnung (20) so konfiguriert ist, dem Zerstäuber (10) Strom zu liefern.

## Revendications

1. Un élément d'atomisation (11), **caractérisé en ce qu'**il comprend :
un substrat (111), comprenant :
une surface d'atomisation (1111) ; et
un film chauffant (112), disposé sur la surface d'atomisation (1111), capable de chauffer et d'atomiser une substance génératrice d'aérosols sur la surface d'atomisation (1111) lorsqu'il est alimenté en énergie, et comprenant :
une couche chauffante métallique (1121) ; et
une couche de protection inorganique (1122), superposée à la couche chauffante métallique (1121), disposée sur la surface de la couche chauffante métallique (1121) éloignée du substrat (111) ;
où la couche chauffante métallique (1121) comprend au moins deux sous-couches métalliques (1121a) empilées successivement, et chacune des deux sous-couches métalliques adjacentes (1121a) présente une composition différente.

2. L'élément d'atomisation (11) selon la revendication 1, dans lequel le substrat (111) est un substrat poreux ; ou bien la couche chauffante métallique (1121) et/ou la couche de protection inorganique (1122) sont des structures poreuses.

3. L'élément d'atomisation (11) selon la revendication 1, dans lequel la couche de protection inorganique (1122) présente des caractéristiques de lipophilie et/ou d'hydrophilie.

4. L'élément d'atomisation (11) selon la revendication 1, dans lequel la couche chauffante métallique (1121) comprend :
une première sous-couche métallique (1121b) ; et
une deuxième sous-couche métallique (1121c), superposée à la première sous-couche métallique (1121b) ;
où le matériau de la première sous-couche métallique (1121b) comprend un alliage NiCr ou un acier inoxydable 316L, et le matériau de la deuxième sous-couche métallique (1121c) comprend un alliage TiZr.

5. L'élément d'atomisation (11) selon la revendication 4, dans lequel la couche chauffante métallique (1121) est en contact direct avec le substrat (111) par l'intermédiaire de la deuxième sous-couche métallique (1121c).

6. L'élément d'atomisation (11) selon la revendication 4 ou 5, dans lequel le nombre des premières sous-couches métalliques (1121b) est d'au moins deux, et le nombre des deuxièmes sous-couches métalliques (1121c) est d'au moins deux.

7. L'élément d'atomisation (11) selon la revendication 4, dans lequel l'épaisseur totale de la première sous-couche métallique (1121b) est comprise entre 1 µm et 20 µm, l'épaisseur totale de la deuxième sous-couche métallique (1121c) est comprise entre 0,5 µm et 5 µm, et l'épaisseur de la couche de protection inorganique (1122) est comprise entre 0,1 µm et 5 µm.

8. L'élément d'atomisation (11) selon la revendication 1, dans lequel la forme du film chauffant (112) est en S, circulaire, elliptique, semi-circulaire ou en anneau ; ou bien la forme de la couche chauffante métallique (1121) est en S, circulaire, elliptique, semi-circulaire ou en anneau.

9. L'élément d'atomisation (11) selon la revendication 1, comprenant en outre deux électrodes (113) reliées électriquement à la couche chauffante métallique (1121), et dans lequel :
l'une des deux électrodes (113) est disposée sur un premier côté de la couche chauffante métallique (1121), l'autre électrode (113) étant disposée sur un second côté de la couche chauffante métallique (1121) opposé au premier côté ; et
la couche de protection inorganique (1122) recouvre une portion de la couche chauffante métallique (1121) située entre les deux électrodes (113).

10. L'élément d'atomisation (11) selon la revendication 9, dans lequel le film chauffant (112) a une forme rectangulaire, l'une des deux électrodes (113) étant disposée sur un premier côté du rectangle, l'autre électrode (113) étant disposée sur un second côté du rectangle opposé au premier ; et un creux (1123) qui ne génère pas de chaleur est défini dans le film chauffant (112).

11. L'élément d'atomisation (11) selon la revendication 10, dans lequel le creux (1123) comprend :
des premiers creux (1123a), en forme d'éventail, dont un premier est situé sur un troisième côté du rectangle, un second des premiers creux (1123a) étant situé sur un quatrième côté du rectangle opposé au troisième côté, et le troisième côté étant perpendiculaire au premier côté du rectangle ; et
un second creux (1123b), situé au centre du film chauffant (112), la forme du second creux (1123b) étant elliptique ou circulaire.

12. L'élément d'atomisation (11) selon la revendication 2, dans lequel une couche de modification est en outre disposée sur la surface d'atomisation (1111) du substrat poreux, et l'épaisseur de la couche de modification est comprise entre 50 µm et 300 µm.

13. L'élément d'atomisation (11) selon la revendication 12, dans laquelle la couche de modification comprend une composition, en parties massiques, de 56 à 67,5 parties de dioxyde de silicium, 12 à 18 parties d'oxyde d'aluminium, et 2,8 à 5,5 parties d'oxyde de lithium.

14. Un atomiseur (10), **caractérisé en ce qu'**il comprend :
une chambre de stockage de liquide (12), configurée pour stocker une substance génératrice d'aérosols ; et
un élément d'atomisation (11) selon l'une quelconque des revendications 1 à 13.

15. Un dispositif d'atomisation électronique, **caractérisé en ce qu'**il comprend :
un ensemble de batteries (20) ; et
un atomiseur (10) selon la revendication 14 ;
dans lequel l'ensemble de batteries (20) est configuré pour alimenter l'atomiseur (10).
